(19) Europäisches Patentamt
European Patent Office
Office européen des brevets

(11) Veröffentlichungsnummer: **0 371 262 B1**

(12) # EUROPÄISCHE PATENTSCHRIFT

(45) Veröffentlichungstag der Patentschrift: **16.03.94**

(51) Int. Cl.[5]: **C07J 19/00**, G01N 33/53, C12Q 1/68, C07J 41/00, C07J 43/00

(21) Anmeldenummer: **89119938.2**

(22) Anmeldetag: **26.10.89**

(54) **Neue Digoxigenin-Derivate und ihre Verwendung.**

(30) Priorität: **27.10.88 DE 3836656**

(43) Veröffentlichungstag der Anmeldung:
**06.06.90 Patentblatt 90/23**

(45) Bekanntmachung des Hinweises auf die Patenterteilung:
**16.03.94 Patentblatt 94/11**

(84) Benannte Vertragsstaaten:
**AT BE CH DE ES FR GB GR IT LI LU NL SE**

(56) Entgegenhaltungen:
**EP-A- 0 173 251**
**EP-A- 0 174 753**
**GB-A- 2 003 480**

**EXPERIENTIA, Band 36, Oktober 1980, Seiten 1141-1143, Basel, CH; N. MONJI et al.: "Ouantification of digoxin by enzyme immunoassay: synthesis of a maleimide derivative of digoxigenin succinate for enzyme coupling"**

(73) Patentinhaber: **BOEHRINGER MANNHEIM GMBH**
**D-68298 Mannheim(DE)**

(72) Erfinder: **Huber, Erasmus, Dr.rer.nat.**
**Bürgerplatz 18**
**D-8046 Garching(DE)**
Erfinder: **Mühlegger, Klaus**
**Römerstrasse 7**
**D-8128 Polling(DE)**
Erfinder: **Von der Eltz, Herbert, Dr.rer.nat.**
**In der Au 21**
**D-8120 Weilheim(DE)**
Erfinder: **Zink, Bruno**
**Seeblickstrasse 4**
**D-8114 Uffing a. Staffelsee(DE)**

(74) Vertreter: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08**
**20**
**D-81635 München (DE)**

**EP 0 371 262 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Digoxigenin-Derivate und ihre Verwendung.

Digoxigenin-Derivate, bei denen das Grundgerüst des Digoxigenins kovalent an ein anderes Molekül oder einen makromolekularen Träger gebunden vorliegt, werden für eine Vielzahl von bioanalytischen Zwecken verwendet. Insbesondere werden solche Digoxigenin-Derivate in immunologischen Tests (Immunoassays) zur im Hinblick auf therapeutische Aspekte wichtigen Bestimmung von kardiotonischen Glycosiden, insbesondere Digoxin, eingesetzt (vgl. G.C. Oliver et al, J.Clin.Invest. 47 (1968), 1035; U.Barbieri und C. Gandolfi, Clin.Chim. Acta 77 (1977), 257; A. Castro und N. Monji, "Immunochemical Methods", Teil B, herausgegeben von J.L. Langone und H.van Vunakis, Academic Press 1981, S. 523; J.A. Hinds et al, Clinical Chemistry 32 (1986) 16). In diesen Tests werden die Digoxigenin-Derivate in Verbindung mit Antikörpern gegen die zu bestimmenden kardiotonischen Glycoside eingesetzt, und der Nachweis erfolgt auf der Grundlage des Prinzips der Hapten-Anti-HaptenAntikörper-Wechselwirkung(vgl. K. Luebke und B. Nieuweboer, "Immunologische Teste für niedermolekulare Wirkstoffe", Thieme Verlag Stuttgart, 1978).

Als Digoxigenin-Derivate werden dabei im allgemeinen solche Derivate verwendet, in denen das Digoxigenin über die 3-Stellung des Steroidgerüstes kovalent an das andere Molekül gebunden ist.

Die in den immunologischen Tests bisher verwendeten Digoxigenin-Derivate weisen jedoch einen oder mehrere der folgenden Nachteile auf:

Bei einer über eine Estergruppierung erfolgenden Verknüpfung mit dem Digoxigenin-Steroidgerüst sind die Digoxigenin-Derivate aufgrund der Hydrolyseempfindlichkeit der Estergruppierung unter basischen Bedingungen sehr basenlabil; dies trifft im besonderen Maße für die üblicherweise verwendeten Hemisuccinate oder Hemiglutarate (vgl. CH-PS 604 164; US-PS 4 082 747; N. Monji et al, Experientia 36 (1980) 1141) zu; ähnliches gilt z.B. auch für die anstelle der Estergruppierung verwendete Urethangruppierung (vgl. DE-OS 26 07 826). Bei der Anwendung kann es deshalb unter bestimmten Bedingungen zu einer unerwünschten Abspaltung des Digoxigenins kommen.

Außer in der 3-Stellung des Steroidgerüstes besitzt Digoxigenin auch in der 12-Stellung eine reaktive OH-Funktion; bei der Herstellung der Derivate fällt deshalb oft ein Gemisch aus 3- und 12-Derivat an, das sich zum Teil nur unter erheblichem präparativen Aufwand in die reinen Stellungsisomeren auftrennen läßt und zum Teil, z.B. im Fall der bevorzugt verwendeten Hemisuccinate und Hemiglutarate, chromatographisch nicht auftrennbar ist. Die Anwendung der nicht aufgetrennten Gemische kann aber zu fehlerhaften Ergebnissen führen.

In den Derivaten liegt das Steroidgrundgerüst zum Teil derart modifiziert vor, daß eine Erkennung des Haptens durch einen gegen das unmodifizierte Steroidgrundgerüst gerichteten Antikörper stark beeinträchtigt sein kann. Dies kann z.B. bei Digoxigenin-Derivaten, in denen das Sauerstoffatom in der 3-Stellung durch Amino-Stickstoff ersetzt ist, der Fall sein (vgl. z.B. EP-B 104 527).

Aufgabe der vorliegenden Erfindung war es, Digoxigenin-Derivate bereitzustellen, die sich gut für immunologische Tests zur Bestimmung von kardiotonischen Glycosiden eignen und mit denen sich die vorstehend beschriebenen Nachteile vermeiden lassen. Diese Aufgabe wird mit der vorliegenden Erfindung gelöst.

Gegenstand der Erfindung sind Digoxigenin-Derivate der allgemeinen Formel (I)

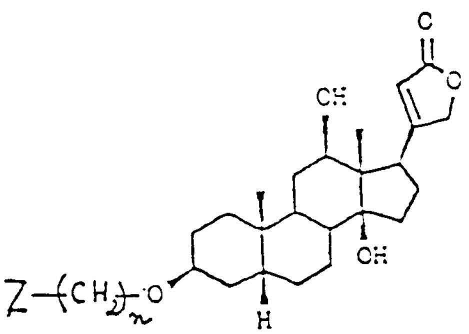

(I)

worin n eine ganze Zahl von 1 bis 4, und vorzugsweise 1, darstellt, und Z die Gruppe -COOH, -CN, $-COOR^5$, $-CONR^1R^2$, $-COONR^3R^4$, $-CON_3$, $-COOCOO-R^5$ oder $-COOCOR^5$ bedeutet, worin $R^1$, $R^2$, $R^3$ und $R^4$ unabhängig voneinander ein Wasserstoffatom, eine Alkylgruppe, eine Aralkylgruppe oder eine Arylgruppe sind, oder $R^1$ und $R^2$ oder $R^3$ und $R^4$ zusammen auch ein carbocyclisches oder heterocyclisches

Ringsystem bilden können.

$R^5$ ist eine Alkyl- oder Aralkylgruppe, vorzugsweise mit 1 bis 7 Kohlenstoffatomen, insbesondere Methyl, Ethyl, Propyl, Isobutyl, Phenyl, Benzyl.

In den vorstehend genannten Resten kann eine Alkylgruppe verzweigt oder vorzugsweise geradkettig sein; vorzugsweise besitzt die Alkylgruppe 1 bis 12, und insbesondere 2 bis 8 Kohlenstoffatome. Eine Alkylgruppe kann durch ein oder mehrere Heteroatome wie z.B. Sauerstoff, unterbrochen sein und substituiert oder unsubstituiert sein.

Eine Arylgruppe kann ein ein- oder mehrkerniger carbo-oder heterocyclischer aromatischer Rest mit vorzugsweise 5 bis 14 Ringatomen sein; sie kann substituiert oder unsubstituiert sein und ist insbesondere Phenyl. Eine Aralkylgruppe leitet sich von den vorstehend definierten Alkyl- und Arylresten ab; sie ist z.B. eine Benzyl-oder Phenylethyl-Gruppe.

Die Gruppierung $-CONR^1R^2$ ist z.B. der Rest $-CONH(CH_2CH_2O)_2CH_2CH_2NH_2$, in dem die $NH_2$-Gruppe substituiert sein kann, vorzugsweise durch 4-Azidobenzoyl. Die Gruppe $-COONR^3R^4$ ist vorzugsweise die N-Hydroxysuccinimidestergruppierung

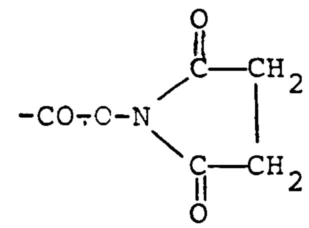

oder die Gruppierungen

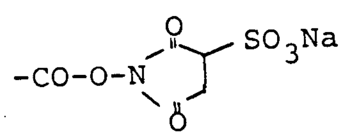
,
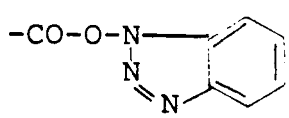
,
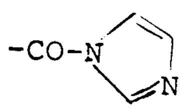

Eine Alkylgruppe $R^5$ ist insbesondere Ethyl, Isobutyl oder Benzyl.

In den erfindungsgemäßen Digoxigenin-Derivaten der Formel (I) erfolgt die Bindung zwischen dem Steroidgerüst und der Brücke über eine Etherbindung in 3-Stellung, wodurch die mit einer Estergruppierung verbundene Basenlabilität vermieden wird. Außerdem wird dadurch die im Digoxigenin-Steroidgerüst in 3-Stellung vorhandene Funktion (Oxy-Gruppe) aufrechterhalten, wodurch eine bessere Erkennung des Haptens durch den Antikörper, der in der Regel gegen das nicht-modifizierte Steroidgerüst gerichtet ist, gewährleistet wird. Die erfindungsgemäße Etherbindung in 3-Stellung ermöglicht auch die Darstellung der Verbindungen der Formel (I) und deren Derivate und Konjugate als reine Stellungsisomere, d.h. unter Vermeidung eines Gemisches aus Derivaten, die in 3- und 12-Stellung gebunden sind.

Die Herstellung der erfindungsgemäßen Verbindungen der Formel (I) kann ausgehend von 12-O-Acetyl-digoxigenin (im nachstehenden Reaktionsschema die Verbindung 3) erfolgen, das aus Digoxin (Verbindung 1) nach Überführung in das Pentaacetyl-digoxin (Verbindung 2) und saure Verseifung auf an sich bekannte Weise erhältlich ist. Im 12-O-Acetyl-digoxigenin wird nun die freie OH-Gruppe in 3-Stellung in die Ethergruppierung $(Z(CH_2)_nO$- überführt. Diese Umsetzung kann mit einem Diazocarbonsäureester, für n = 1 z.B. mit Diazoessigester, auf an sich bekannte Weise unter Bildung des 3-Alkoxycarbonylalkylethers erfolgen ($Z = COOR^5$; entspricht Verbindung 4). In dem erhaltenen Alkoxycarbonylalkylester kann, wenn erwünscht, die Estergruppierung durch Verseifung in die freie Carboxygruppe ($Z = COOH$; Verbindung 5) oder eine andere der genannten Carbonsäuregruppierungen Z überführt werden, und, wenn erwünscht, kann in dem so erhaltenen Reaktionsprodukt die Gruppe Z auf an sich bekannte Weise in eine andere Gruppe Z gemäß Formel (I) überführt werden. Durch Umsetzung mit N-Hydroxysuccinimid kann die COOH-Gruppe z.B. in die Gruppierung $-COONR^3R^4$ ($R^3$ und $R^4$ bilden zusammen den 1,4-Dioxo-tetramethylen-Rest; Verbindung 6) überführt werden; diese Gruppierung läßt sich durch Umsetzung mit einem Amin

$HNR^1R^2$ in die Verbindung (I) mit Z = $CONR^1R^2$ (entspricht Verbindung 7) überführen. Auf diese oder ähnliche, an sich bekannte Weise ist es möglich, eine erhaltene Verbindung der Formel (I), in der Z eine der angegebenen Bedeutungen besitzt, in eine andere Verbindung der Formel (I) mit einer anderen Bedeutung von Z zu überführen.

Die einzelnen, vorstehend genannten Verfahrensschritte, wie z.B. die Umsetzung mit Diazoessigester, die Verseifungsstufen, die Umsetzung mit N-Hydroxysuccinimid und die Umsetzung mit einem Amin $HNR^1R^2$ kann auf eine für diese Umsetzung übliche Weise erfolgen, wie z.B. in den nachfolgenden Beispielen erläutert.

Ein weiteres wichtiges Einsatzgebiet für Digoxigenin-Derivate ist auch ihre Verwendung zur Markierung und Detektion von Nucleinsäuren und Nucleinsäurederivaten. In den deutschen Patentanmeldungen der gleichen Anmelderin P 38 00 644.8 (vom 12. Januar 1988) und P 38 13 278.8 (vom 20. April 1988) wird ein Verfahren zum Nachweis von Nucleinsäuren durch Hybridisierung mit einer komplementären Nucleinsäure-Sonde, die über eine chemische Verbindung mindestens ein Hapten als Markierung gebunden enthält, beschrieben. Als Hapten verwendet man ein Steroid, das an mindestens eine Position der Nucleinsäure-Sonde, die nicht an der Wasserstoffbrückenbildung beteiligt ist, über eine Brücke von mindestens 4 Atomen Länge gebunden ist; die hybridisierte Sonde weist man über einen markierten Anti-Hapten-Antikörper nach. Als Steroid wird vorzugsweise Digoxigenin oder Digoxin verwendet. Wird das Hapten in die Nucleinsäure-Sonde fotochemisch mit Hilfe eines Foto-Haptens eingebaut (vgl. Nucl.Acid Res. 13 (1985) 745 bis 761; und M.Wilchek und E.A. Bayer, Anal. Biochem. 171 (1988) 1), so verwendet man als Foto-Hapten vorzugsweise Foto-Digoxigenin, d.h. ein über eine Brücke mit dem 4-Azido-benzoylrest verbundenes Digoxigenin. Bei UV-Bestrahlung entsteht unter Abspaltung von Stickstoff aus der Azidogruppe ein Nitrenradikal, das dann kovalent an die Nucleinsäure bindet. Als Foto-Digoxigenin wird das Digoxigenin-3-hemisuccinat-[N′-(4-azidobenzoyl)]-8-amino-3,6-dioxaoctylamid verwendet.

Aufgrund ihrer Eigenschaften, insbesondere aufgrund der Basenstabilität und des unmodifizierten Steroidgerüstes, eignen sich die erfindungsgemäßen Digoxigenin-Derivate der Formel (I) sehr gut als Markierungshapten für das vorstehend genannte Verfahren zum Nachweis von Nucleinsäuren. Als Foto-Hapten (Foto-Digoxigenin; Bindung des Haptens auf fotochemischem Wege, vgl. Nucl. Acid Res. 13 (1985) 745 bis 761; und M.Wilchek und E.A.Bayer, Anal. Biochem. 171 (1988) 1) wird ein erfindungsgemäßes Digoxigenin-Derivat der Formel (I) verwendet, worin einer der Reste $R^1$, $R^2$, $R^3$, $R^4$ und/oder $R^5$ eine 4-Azidobenzoyl-Gruppe trägt, wie z.B. das N-[N-(4-Azidobenzoyl)-8-amino-3,6-dioxaoctyl]-3-carbamoylmethyl-digoxigenin (Verbindung 7 des Reaktionsschemas).

Mit den erfindungsgemäßen Digoxigenin-Derivaten der allgemeinen Formel (I) ist es möglich, die 3-Position des Steroids mit dem dort vorhandenen Sauerstoff, also ohne Modifizierung des Grundgerüstes, über ein Brückenmolekül an ein immunogenes Trägermaterial, wie ein Protein- oder Polypeptid-Trägermaterial, oder an eine Nucleinsäure zu binden.

Die erfindungsgemäßen Digoxigenin-Derivate der Formel (I) eignen sich auch sehr gut zur Herstellung von markierten Konjugaten (labeled conjugates), wie sie zur Bestimmung von kardiotonischen Glycosiden, insbesondere von Digoxin, in üblichen Immunoassays verwendet werden. Geeignete Konjugate können z.B. in Übereinstimmung mit Standardmethoden radioaktiv markiert oder mit einer Fluoreszenz-Gruppierung markiert sein. Die markierte Struktureinheit (labeling moiety) ist in den bevorzugten homogenen Techniken z.B. ein Enzymsubstrat, eine prostetische Gruppe, ein Enzymmodulator oder ein Enzym, das mit den erfindungsgemäßen Digoxigenin-Derivaten der Formel (I) zum Konjugat gekuppelt ist.

Die Bindung an das Trägermaterial, an die Nucleinsäure oder an die markierte Struktureinheit erfolgt über die Gruppierung $-O-(CH_2)_n-Z$, wobei die Gruppierung Z vorzugsweise abhängig vom Trägermaterial und den mit Z reagierenden Gruppen oder Bindungen ausgewählt wird. Vorzugsweise erfolgt die Verknüpfung des Trägermaterials mit der $-O-(CH_2)_n-Z$-Gruppierung über primäre oder sekundäre Aminogruppen, die mit einer aktivierten Carbonsäurefunktion Z umgesetzt werden, z.B. mit dem N-Hydroxysuccinimid-ester (Z = $-COONR^3R^4$, $R^3$ und $R^4$ bilden zusammen den 1,4-Dioxotetramethylen-Rest); die Umsetzung erfolgt dabei auf für eine solche Reaktion an sich bekannte Weise. Die Gruppierung $-O-(CH_2)_n-Z$ kann aber auch nach der fotochemischen Methode (vgl. Nucl.Acid Res. 13 (1985) 745 bis 761; und M.Wilchek und E.A. Bayer, Anal.Biochem. 171 (1988) 1) an den Träger, z.B. die Nucleinsäure, gebunden werden; in diesem Fall wird das Trägermaterial, z.B. die Nucleinsäure-Sonde, in Gegenwart des vorstehend genannten Foto-Digoxigenins (siehe Reaktionsschema, Verbindung 7) mit sichtbarem Licht mit UV-Anteil bestrahlt, wobei unter Abspaltung von Stickstoff ($N_2$) ein Nitrenradikal entsteht, das kovalent an die Nucleinsäure bindet.

Gegenstand der Erfindung ist deshalb auch die Verwendung von Digoxigenin-Derivaten der allgemeinen Formel (I) zur Herstellung von markierten Konjugaten (labeled conjugates) zur Bestimmung von kardiotonischen Glycosiden, insbesondere von Digoxin, in üblichen Immunoassays und ihre Verwendung als Markerhapten zum Nachweis von Nucleinsäuren durch Hybridisierung mit einer komplementären Nucleinsäure-Sonde, die über eine chemische Verbindung mindestens ein Hapten als Markierung gebunden enthält.

Ein weiterer Gegenstand der Erfindung sind auch die unter Verwendung der erfindungsgemäßen Digoxigenin-Derivate der Formel (I) hergestellten neuen Digoxigenin-Konjugate der Formel (II)

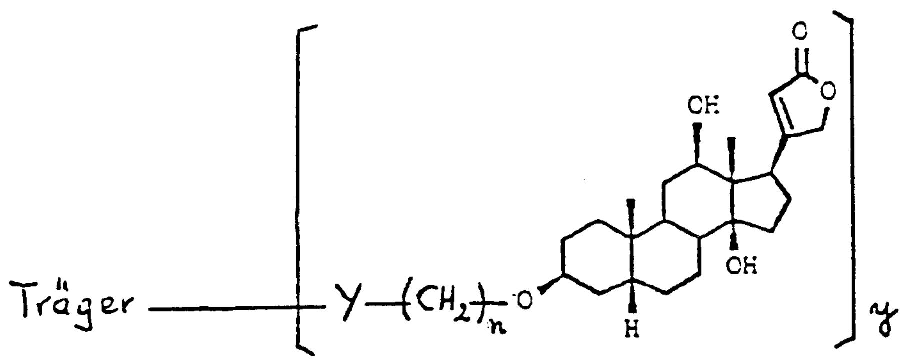

(II)

worin der Träger ein immunogenes Trägermaterial, wie z.B. ein immunogenes Protein- oder Polypeptid-Trägermaterial, eine Nucleinsäure oder die markierte Struktureinheit eines markierten Digoxigenin-Konjugates für die Verwendung in Immunoassays bedeutet, y im Durchschnitt eine Zahl von 1 bis zu der Zahl der im Träger verfügbaren Kupplungsstellen bedeutet, und vorzugsweise 1 bis 20 ist, und Y die durch Umsetzung der Carboxylfunktion Z der erfindungsgemäßen Digoxigenin-Derivate der Formel (I) mit den reagierenden Stellen des Trägermaterials gebildete Gruppierung ist, z.B. eine Amidgruppe.

Weiterer Gegenstand der Erfindung ist auch die Verwendung der Digoxigenin-Konjugate der Formel (II), worin der Träger ein Immunogen darstellt, zur Immunisierung von zur Antikörper-Bildung geeigneten Organismen. Auf diese Weise können mit den erfindungsgemäßen Digoxigenin-Konjugaten der Formel (II), worin der Träger ein Immunogen darstellt, Antikörper hergestellt werden, die dann in einem der üblichen Immunoassay-Verfahren zur Bestimmung von Digoxin (z.B. Agglutinationstechniken, Radioimmunoassays, heterogene Enzym-Immunoassays, heterogene Fluoreszenz-Immunoassays, und homogene Immunoassays) eingesetzt werden können. Die Herstellung und Isolierung dieser Antikörper, einschließlich monoklonaler Antikörper kann auf eine dafür übliche und allgemein bekannte Weise erfolgen.

Ein weiterer Gegenstand ist auch die Verwendung der Digoxigenin-Konjugate der Formel (II), in denen der Träger die markierte Struktureinheit eines markierten Digoxigenin-Konjugates darstellt, in üblichen Immunoassays zur Bestimmung von kardiotonischen Glycosiden, insbesondere von Digoxin.

Die nachfolgenden Beispiele sollten die Erfindung näher erläutern, ohne sie darauf zu beschränken. Wenn nicht anders angegeben, beziehen sich die Mengenangaben auf Gewichtsteile und Temperaturangaben auf °C. Unter Raumtemperatur wird eine Temperatur von 25 ± 2 °C verstanden.

## Beispiele

Das nachfolgende Reaktionsschema A gibt einen Überblick über die in den Beispielen gezeigte Synthese der erfindungsgemäßen Digoxigenin-Derivate der Formel (I) und deren gegenseitige Überführung.

Reaktionsschema A

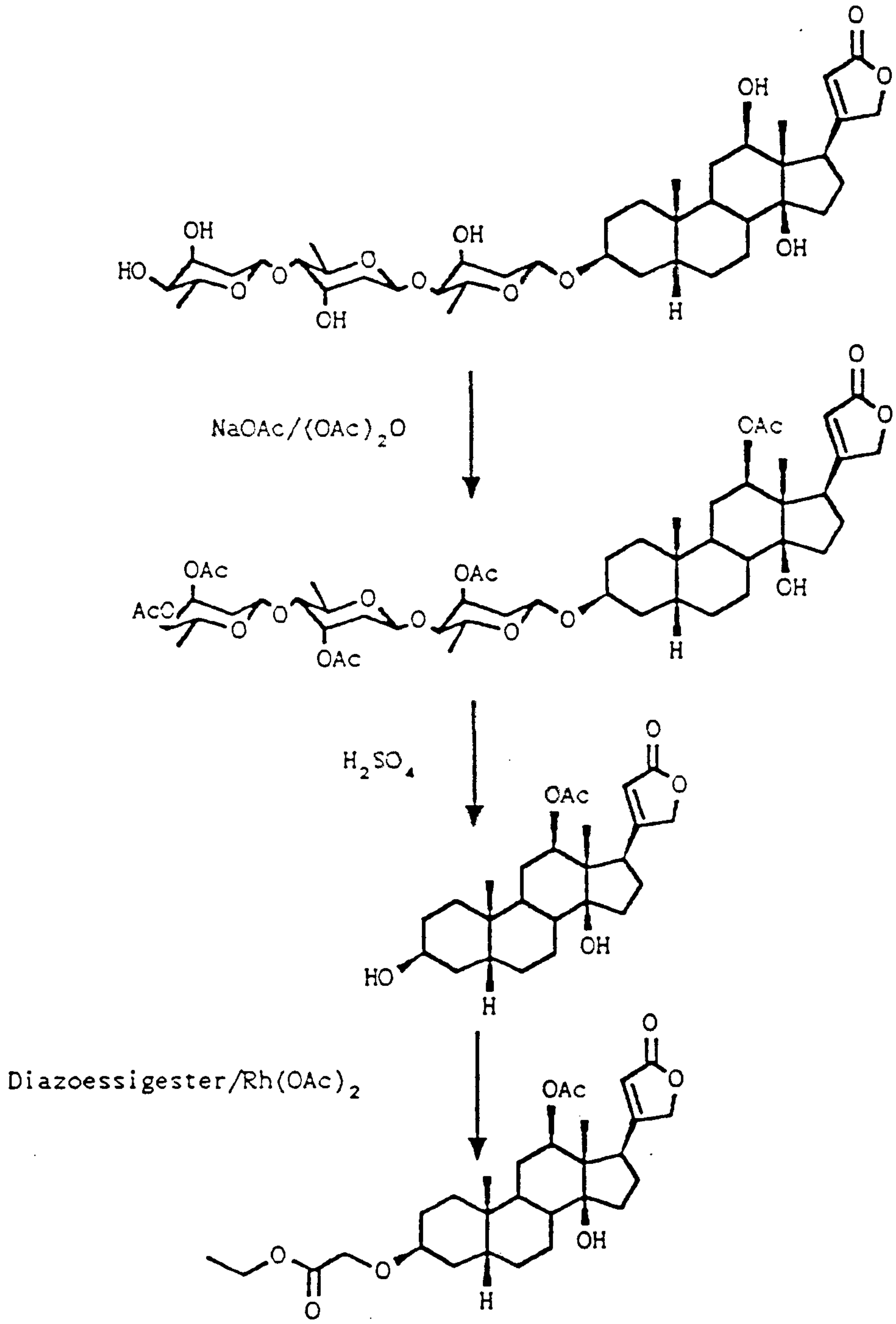


1

2

3

4

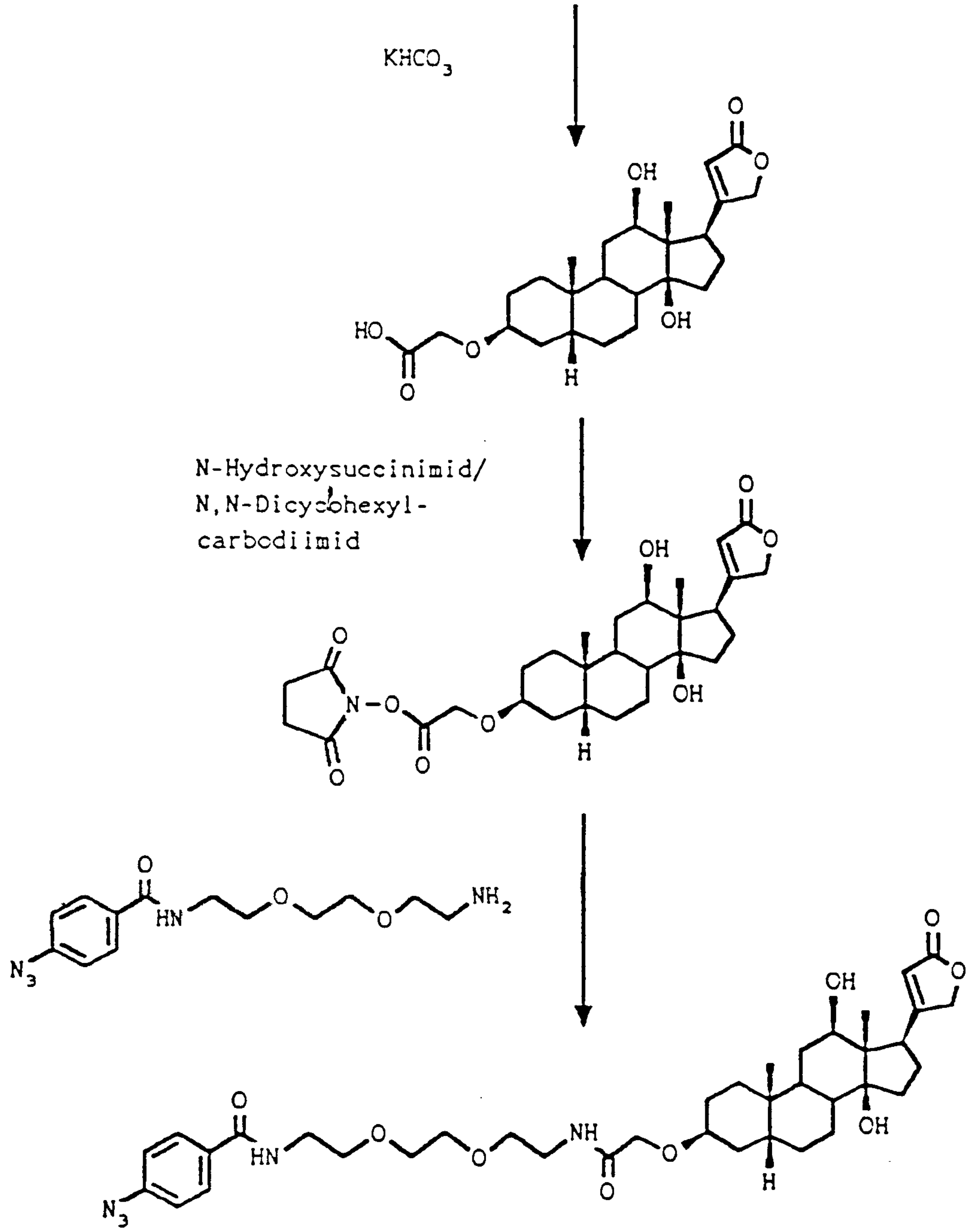


5

6

7

Beispiel 1: Herstellung von Pentaacetyl-digoxin (2)

78 g (0,1 mol) Digoxin werden in 1 l Acetanhydrid gelöst und mit 49,2 g (0,6 mol) Natriumacetat (wasserfrei) versetzt. Man läßt 1 Stunde unter Rückfluß rühren. Dann dampft man die Lösung am Wasserstrahlvakuum ein, löst den Rückstand in 1 l Essigester und filtriert von eventuell unlöslichen Bestandteilen ab. Das Filtrat wird dreimal mit je 0,5 l Wasser gewaschen, mit 50 g $Na_2SO_4$ getrocknet und im Wasserstrahlvakuum eingedampft. Das Rohprodukt enthält noch Acetanhydrid.

Ausbeute: 110 g zähes Öl.

DC: Kieselgel, Essigester/Chloroform 1:1; $R_f$ = 0,33.

Beispiel 2: Herstellung von 12-O-Acetyl-digoxigenin (3)

Der nach Beispiel 1 erhaltene Eindampfrückstand (Verbindung 2) wird in 2 l Methanol gelöst, mit 2 l 0,1 n $H_2SO_4$ versetzt und 1 Stunde unter Rückfluß geruhrt. Danach wird einmal mit 1,8 l und einmal mit 600 ml Chloroform extrahiert, die vereinigten Extrakte zweimal mit je 1 l Wasser gewaschen, mit 50 g $Na_2SO_4$ getrocknet und am Wasserstrahlvakuum eingedampft. Das erhaltene Öl (95 g) wird unter leichtem Erwärmen in 250 ml Essigester gelöst und bei Raumtemperatur stehengelassen. Nach kurzer Zeit tritt Kristallisation ein. Man läßt etwa 4 Stunden bei Raumtemperatur und weitere 2 Stunden bei +4°C kristallisieren, dann saugt man den Feststoff ab und wäscht kurz mit ca. 100 ml Essigester.

Ausbeute: 31 g farblose Kristalle.

DC: Kieselgel, Essigester; $R_f$ = 0,50.

Beispiel 3: Herstellung von 12-O-Acetyl-digoxigenin-3-cme-ethylester (4)

28,1 g (65 mmol) der nach Beispiel 2 erhaltenen Verbindung (3) werden in 250 ml Tetrahydrofuran (THF) suspendiert und im Verlauf von 5 Stunden 69 ml (0,65 mol) Diazoessigester in 50 ml THF unter Rühren zugetropft. Zum Reaktionsstart und nach jeweils 1 Stunde werden je 50 mg Rhodium(II)-acetat zugegeben. Da dabei eine leichte Erwärmung der Reaktionslösung auftritt, empfiehlt es sich, die Lösung mit einem Wasserbad (25°C) zu kühlen. Man läßt 16 Stunden bei Raumtemperatur rühren, dann werden nach den oben beschriebenen Verfahren nochmals 69 ml Diazoessigester und insgesamt 250 g Rhodiumacetat zugegeben. Nach weiteren 16 Stunden wird der gesamte Vorgang ein drittes Mal wiederholt. Man läßt anschließend noch 1 Tag nachreagieren, versetzt dann mit 250 ml Methanol und dampft die Lösung im Vakuum ein. Der ölige Rückstand wird dreimal mit je 1 l Petrolether digeriert und nach Dekantieren in 100 ml Chloroform/Essigester = 2/1 gelöst. Man gibt das Rohprodukt auf eine Kieselgel-Säule (8,5 x 50 cm) und eluiert mit Chloroform/Essigester = 2/1. Die das reine Produkt (4) enthaltenden Fraktionen werden vereinigt, und das Lösungsmittel im Wasserstrahlvakuum entfernt.

Ausbeute: 18,2 g zähes Öl.

DC: Kieselgel, Essigester/Petrolether 2:1, $R_f$ = 0,60.

Beispiel 4: Herstellung von Digoxigenin-3-carboxymethylether (cme) (5)

15,5 g (30 mmol) der nach Beispiel 3 erhaltenen Verbindung (4) löst man in 470 ml Methanol und versetzt mit einer Lösung von 6,05 g (60 mmol) $KHCO_3$ in 100 ml Wasser. Man läßt unter Rückfluß rühren und kontrolliert den Umsatz halbstündlich mittels DC. Wenn die Konzentration des Edukts nur noch ca. 5 % beträgt (etwa nach 3,5 Stunden), wird die Reaktion abgebrochen. Man stellt den pH mit Eisessig auf 5,0, dampft das Methanol im Wasserstrahlvakuum ab und verdünnt mit Wasser auf ca. 500 ml. Das Rohprodukt wird mit zweimal je 200 ml Essigester extrahiert, mit 200 ml Wasser gewaschen und die organische Lösung mit 30 g $Na_2SO_4$ getrocknet. Nach dem Eindampfen löst man die verbleibenden 11 g Öl in 40 ml Essigester/Eisessig = 9/1 und läßt bei Raumtemperatur stehen. Nach kurzer Zeit tritt Kristallisation ein. Man kühlt noch 1 Stunde bei +4°C, saugt den Feststoff ab und wäscht mit ca. 20 ml Essigester/Eisessig = 9/1 nach. Man erhält 3 g Produkt (5), das im Exsikkator über KOH getrocknet wird.

Die Mutterlauge wird eingedampft und der Rückstand (ca. 7,5 g) auf eine Kieselgel-Säule (8,5 x 40 cm) aufgetragen. Nach Elution mit Essigester/Eisessig = 9/1 und Eindampfen der entsprechenden Fraktionen werden nochmals 2,2 g Produkt (5) erhalten, das noch Spuren von 12-O-Acetyl-digoxigenin-3-cme enthalten kann.

Ausbeute: 5,2 g farbloser Feststoff.

DC: Kieselgel, Essigester/Eisessig 9:1, $R_f$ = 0,42.

Beispiel 5: Herstellung von Digoxigenin-3-cme-O-succinimid (6)

4,49 g (10 mmol) der nach Beispiel 4 erhaltenen Digoxigenin-Carbonsäure (5) werden zusammen mit 1,27 g (11 mmol) N-Hydroxysuccinimid in 140 ml wasserfreiem THF gelöst und mit einer Lösung von 2,27 g (11 mmol) N,N'-Dicyclohexylcarbodiimid in 20 ml wasserfreiem THF versetzt. Man läßt 20 Stunden bei Raumtemperatur rühren, dann filtriert man vom ausgefallenen Harnstoff ab und dampft die Lösung im Wasserstrahlvakuum ein. Den Rückstand löst man in 150 ml Essigester, filtriert und wäscht mit 100 ml Wasser. Die organische Lösung wird danach sofort mit 5 g $Na_2SO_4$ getrocknet und eingedampft. Man löst das Rohprodukt in ca. 30 ml Essigester, filtriert und gießt unter Rühren langsam in 200 ml Diisopropylether. Der ausgefallene Ester (6) wird abgesaugt und im Exsikkator über Phosphorpentoxid getrocknet.

Ausbeute: 5,1 g farbloses Pulver.
DC: Kieselgel RP-18, Nitromethan/Ethanol 9:1; $R_f$ = 0,66.

Beispiel 6: Herstellung von Photodigoxigenin (7)

272 mg (0,5 mmol) des nach Beispiel 5 erhaltenen Aktivesters (6) werden in 10 ml Dioxan gelöst und mit einer Lösung von 161 mg (0,55 mmol) N-(4-Azidobenzoyl)-1,8-diamino-3,6-dioxaoctan in 5 ml Wasser versetzt. Man läßt 2 Stunden bei Raumtemperatur rühren, dann dampft man das Dioxan im Wasserstrahlvakuum ab und verdünnt mit 50 ml Wasser. Die wäßrige Phase wird mit zweimal je 50 ml Essigester extrahiert, die vereinigten organischen Extrakte mit 5 g $Na_2SO_4$ getrocknet und eingedampft. Den Rückstand digeriert man mit 100 ml Diisopropylether, saugt ab und trocknet im Exsikkator über $CaCl_2$.

Ausbeute: 216 mg farbloses Pulver.
DC: Kieselgel RP-18, Nitromethan/Ethanol 9:1; $R_f$ = 0,36.

Das folgende Reaktionsschema B zeigt schematisch die schrittweise Herstellung von Dig-11-dUTP, das z.B. als Markerhapten zum Nachweis von Nukleinsäuren durch Hybridisierung mit einer komplementären, markierten Nukleinsäure verwendet werden kann, gemäß den Beispielen 7 bis 9.

Reaktionsschema B

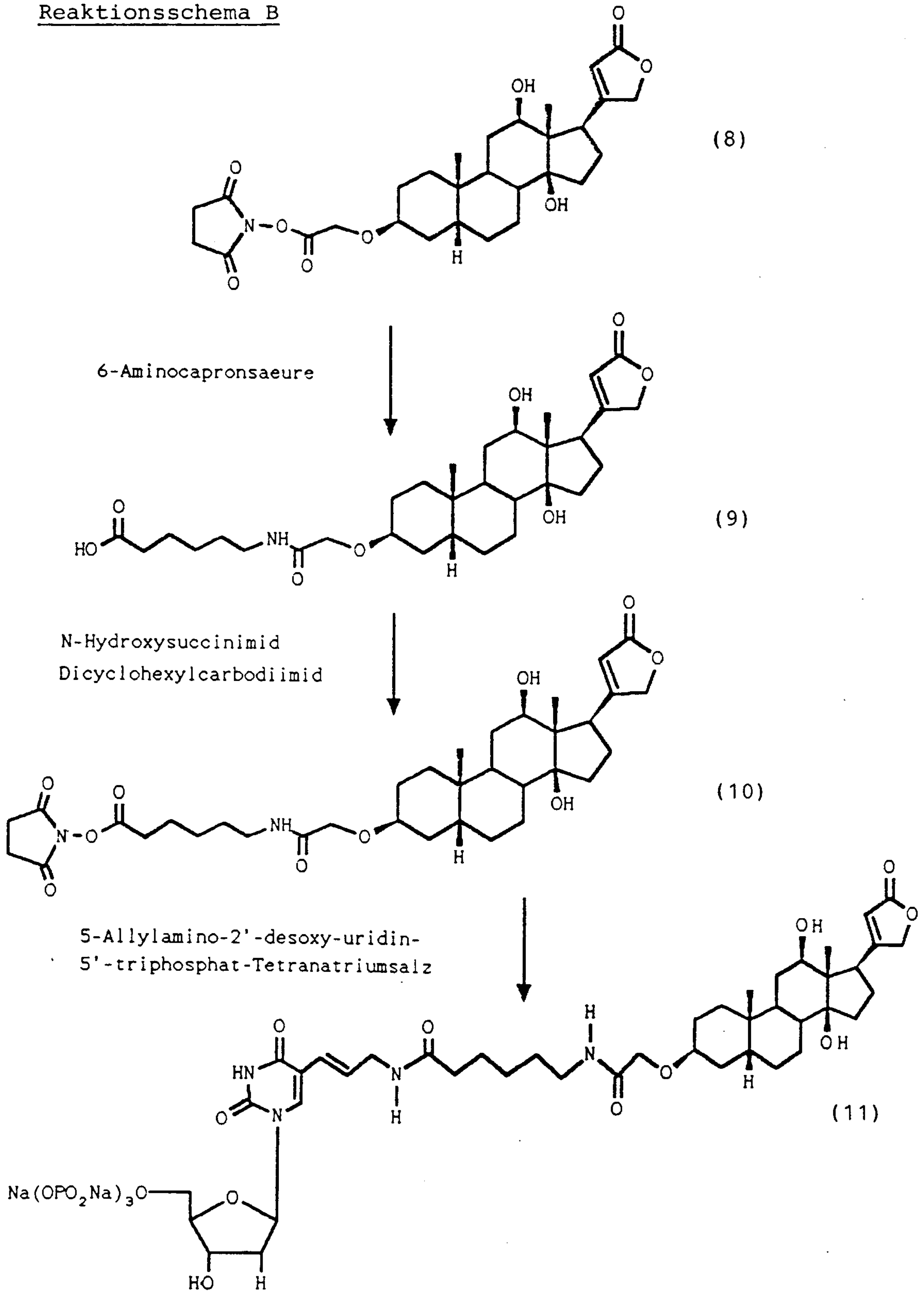


(8)

(9)

(10)

(11)

Beispiel 7: Digoxigenin-3-carboxymethylether- $\epsilon$ -amidocapronsäure (9)

$C_{31}H_{47}NO_8$ MG: 561.3

In einem 250 ml-Rundkolßen werden 465 mg Digoxigenin-3-carboxymethylether-N-hydroxysuccinimidester (8) (0,85 mmol) in 15 ml Dimethylformamid (DMF) gelöst und dazu eine Suspension von 112 mg 6-Aminocapronsäure (0,85 mmol) und 0,12 ml Triethylamin in 2 ml DMF gegeben. Man rührt über Nacht bei Raumtemperatur magnetisch, wobei allmählich eine homogene Lösung entsteht. Nach dieser Zeit ist die Umsetzung laut Dünnschichtchromatographie (Kieselgel; Essigsäureethylester/Petrolether/Ethanol 1:1:1, Detektion: Besprühen mit einer Mischung von 10 ml Eisessig + 0,2 ml konz. $H_2SO_4$ + 0,1 ml Anisaldehyd und Erhitzen auf 120 °C bis zum Erscheinen blauschwarzer Flecke; $R_f$ ca. 0,7; $R_f$ Digoxigenin-OSu-ester ca. 0,85) praktisch vollständig.

Man destilliert DMF im Hochvakuum restlos ab und löst das verbleibende Öl in 5 ml $H_2O$ unter Zugabe von konz. Ammoniaklösung. Dann wird durch Zufügen von 22,5 ml wäßriger Zitronensäurelösung (100 g Zitronensäure/l) die "freie" Digoxigeninamidocapronsäure abgeschieden. Die harzig-zähe Masse wird durch Anreiben mit Wasser fest; man saugt ab, wäscht mehrfach mit $H_2O$ nach und trocknet letztlich über $P_2O_5$ im Ölpumpenvakuum.

Ausbeute: 325 mg = 68 % der Th.

Beispiel 8: Digoxigenin-3-carboxymethylether- $\epsilon$ -amidocapronsäure-N-hydroxysuccinimidester (10)

$C_{35}H_{50}N_2O_{10}$ MG: 658.8

In einem 100 ml-Rundkolben werden 320 mg Digoxigenin-3-carboxymethylether- $\epsilon$ -amidocapronsäure (9) (0,57 mmol) in 2 ml wasserfreiem Dimethylformamid (DMF) gelöst, und nacheinander mit 70 mg N-Hydroxysuccinimid (0,6 mmol), sowie 130 mg Dicyclohexylcarbodiimid (0,63 mmol) versetzt. Man rührt über Nacht bei Raumtemperatur, saugt anderntags vom abgeschiedenen Dicyclohexylharnstoff ab und zieht das DMF im Ölpumpenvakuum ab. Das zurückbleibende Öl wird in 2 ml Essigsäureethylester aufgenommen und in ca. 15 ml eiskalten (-20 °C) Petrolether eingerührt. Das ausgefallene, anfangs noch harzig-zähe Produkt reibt man mehrfach mit eiskaltem trockenem Petrolether bis zum Festwerden durch. Nach Trocknung über $P_2O_5$ im Vakuum erhält man 315 mg = 84 % der Th.

| Elementaranalyse: | | |
|---|---|---|
| C ber.: 63,8 %<br>C gef.: 63,2 % | H ber.: 7,6 %<br>H gef.: 7,6 % | N ber.: 4,2 %<br>N gef.: 4,0 % |

Beispiel 9: Digoxigenin-3-carboxymethylether- $\epsilon$ -amidocaproyl-[5-(amidoallyl)-2′-desoxy-uridin-5′-triphosphat]-Tetranatriumsalz (11)

(Dig-11-dUTP)

$C_{43}H_{61}N_4Na_4O_{21}P_3$ MG: 1154,7

245 mg Digoxigenin-3-carboxymethylether- $\epsilon$ -amidocapronsäure-N-hydroxysuccinimidester (10) (0,37 mmol) werden in 7 ml DMF gelöst und zu einer Lösung von 20 mg 5-Allylamino-2′-desoxy-uridin-5′-triphosphat-Tetralithiumsalz (0,37 mmol) in 6 ml $H_2O$ gegeben. Man fügt dem Gemisch 6,2 ml 0,1 mol/l Natriumboratpuffer, pH 8,5 zu und rührt bei Raumtemperatur über Nacht (ca. 15 Stunden).

In der Papierelektrophorese (0,05 mol/l Citratpuffer, pH 5,0) beobachtet man im UV-Licht nach dieser Zeit neben etwas unumgesetztem Allylamino-dUTP einen etwas tiefer laufenderen Fleck der gewünschten Verbindung (alternativ: Dünnschichtchromatographie (DC) auf Kieselgel, Fließmittel Isobuttersäure/konz. Ammoniaklösung/$H_2O$ = 66:1:33, Detektion im UV oder Besprühen mit Anisaldehyd-Reagenz - siehe Beispiel 7 -; $R_f$-Werte: 5-Allylamino-dUTP 0,2; Dig-amidocapronsäure-OSu-ester 0,7; Dig-11-dUTP 0,45).

Zur Aufreinigung wird das Reaktionsgemisch im Ölpumpenvakuum bis zum festen Rückstand eingedampft, in 200 ml $H_2O$ aufgenommen und auf eine Ionenaustauschersäule (DEAE-Sephadex A25, $HCO_3^-$-Form, Säulendimension 1,5 x 30 cm) gegeben. Nach Aufziehen wird kurz mit Wasser gewaschen, dann mit einem Gradient von je 1 l $H_2O$ auf 0,4 mol/l TEAB (Triethylammoniumbicarbonat), pH 8 eluiert. Die reines Produkt enthaltenden Fraktionen werden vereinigt, im Vakuum konzentriert und durch mehrfaches Eindampfen mit Methanol von überschüssigem TEAB befreit (kein Geruch von freiem Triethylamin mehr!). Man nimmt den Kolbeninhalt in wenigen ml Wasser auf, passiert die Lösung über eine kurze Kationenaustauschersäule DOWEX 50 WS8 (1 x 10 cm) in der $Na^+$-Form, wäscht die Säule bis zur ODE-Freiheit des Waschwassers Messung im UV bei 240 nm) und dampft im Vakuum bis auf ca. 20 ml ein. Nach Lyophilisation werden 195 mg (45 % der Th.) Dig-11-dUTP-$Na_4$ als weißes Pulver erhalten.
Analytik: $H_2O$-Bestimmung: 7,9 %

Elementaranalyse: (unter Berücksichtigung des $H_2O$-Gehaltes):

| | | | |
|---|---|---|---|
| C ber.: 41,2 % | H ber.: 5,3 % | N ber.: 4,4 % | P ber.: 7,4 % |
| C gef.: 41,0 % | H gef.: 5,4 % | N gef.: 4,6 % | P gef.: 7,2 % |

UV-Spektrum (Phosphatpuffer pH 7,0): Maxima: 220 nm, 290 nm.

Dig-11-dUTP kann nach der "Random primed"-DNA-Markierungsmethode (DNA-Labeling and Detection Kit Nonradioactive, Best.Nr. 1093657, Fa. Boehringer Mannheim; Feinberg, A.P. und Vogelstein, B., Anal. Biochem. 132, (1983) 6) in eine Nukleinsäure eingeführt werden, wobei eine Nukleinsäuresonde, die Digoxigenin als Markierungshapten enthält, zum Nachweis dazu komplementärer Nukleinsäuren erhalten wird.

## Patentansprüche

**Patentsprüche für folgende Vertragsstaaten : AT, BE, CH, LI, DE, FR, GB, GR, IT, LU, NL, SE**

**1.** Digoxigenin-Derivate der allgemeinen Formel (I)

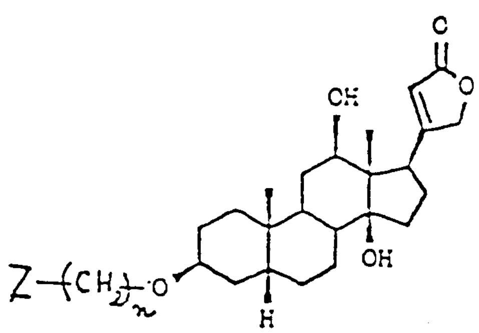

(I)

worin n eine ganze Zahl von 1 bis 4 darstellt, und Z die Gruppe -COOH, -CN, $-COOR^5$, $-CONR^1R^2$, $-COONR^3R^4$, $-CON_3$, $-COOCOO-R^5$ oder $-COOCO-R^5$ bedeutet, worin $R^1$, $R^2$, $R^3$ und $R^4$ unabhängig voneinander ein Wasserstoffatom, eine Alkylgruppe, eine Aralkylgruppe oder eine Arylgruppe sind, oder $R^1$ und $R^2$ oder $R^3$ und $R^4$ zusammen auch ein carbo- oder heterocyclisches Ringsystem bilden können, und $R^5$ eine Alkyl- oder Aralkylgruppe ist.

**2.** Digoxigenin-Derivate der Formel (I) nach Anspruch 1,
**dadurch gekennzeichnet,**
daß Z bedeutet: -COOH, $-COOC_2H_5$,

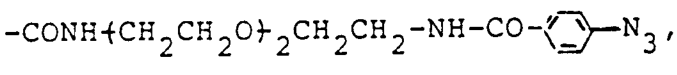 , 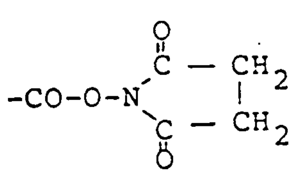

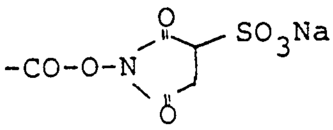,

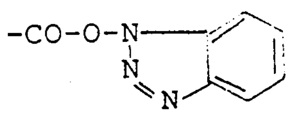,

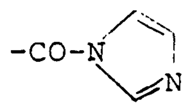

3. Digoxigenin-Derivate der Formel (I) nach einem der Ansprüche 1 oder 2,
**dadurch gekennzeichnet,**
daß n = 1 ist.

4. Verfahren zur Herstellung der Digoxigenin-Derivate der Formel (I) nach einem der Ansprüche 1 bis 3,
**dadurch gekennzeichnet,**
daß man im 12-O-Acetyl-digoxigenin die frei OH-Gruppe in 3-Stellung durch Umsetzung mit Diazoessigester in an sich bekannter Weise in eine Verbindung der Formel (I) überführt, worin Z = $COOC_2H_5$ ist, und, wenn erwünscht, in dem erhaltenen Reaktionsprodukt in an sich bekannter Weise die $COOC_2H_5$-Gruppe in die COOH-Gruppe oder in eine der anderen Gruppen überführt, die Z darstellen kann und, wenn erwünscht, in den so erhaltenen Reaktionsprodukten die Gruppe Z in an sich bekannter Weise in eine andere Gruppe Z gemäß Formel (I) überführt.

5. Verwendung der Digoxigenin-Derivate der Formel (I) gemäß einem der Ansprüche 1 bis 3 zur Herstellung von markierten Konjugaten (labeled conjugates) zur Bestimmung von kardiotonischen Glycosiden, insbesondere von Digoxin, in Immunoassays.

6. Verwendung der Digoxigenin-Derivate der Formel (I) gemäß einem der Ansprüche 1 bis 3 als Markerhapten zum Nachweis von Nucleinsäuren durch Hybridisierung mit einer komplementären Nucleinsäure-Sonde, die über eine chemische Verbindung mindestens ein Hapten als Markierung gebunden enthält.

7. Digoxigenin-Konjugate nach der Formel (11)

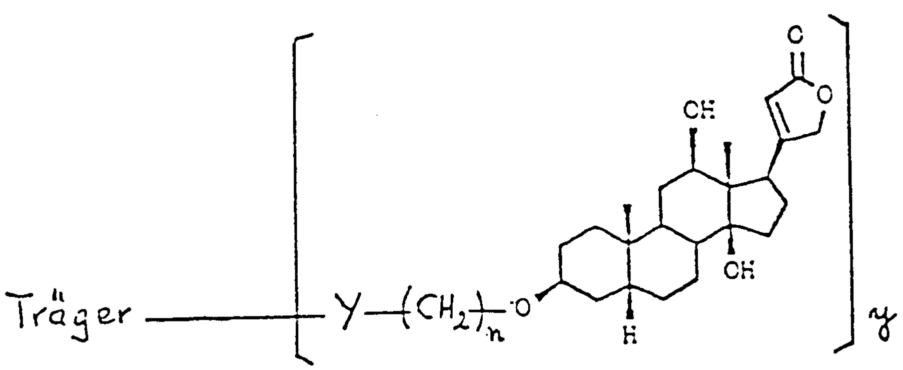

(II)

worin der Träger ein immunogenes Trägermaterial, eine Nukleinsäure oder die markierte Struktureinheit eines markierten Digoxigenin-Konjugates für die Verwendung in Immunoassays bedeutet, y im Durchschnitt eine Zahl von 1 bis 20 bedeutet, und Y die durch Umsetzung der Carboxylfunktion Z der Digoxigenin-Derivate der Formel (I) nach einem der Ansprüche 1 bis 3 und den reagierenden Stellen des Trägermaterials gebildete Gruppierung ist.

8. Verwendung der Digoxigenin-Konjugate der Formel (11) nach Anspruch 7, worin der Träger ein immunogenes Trägermaterial darstellt, zur Immunisierung von zur Antikörper-Bildung geeigneten Organismen.

9. Verwendung der Digoxigenin-Konjugate der Formel (II) nach Anspruch 9, worin der Träger die markierte Struktureinheit eines markierten Digoxigenin-Konjugates darstellt, in Immunoassays zur Bestimmung von kardiotonischen Glycosiden, insbesondere von Digoxin.

**Patentansprüche für folgenden Vertragsstaat : ES**

1. Verfahren zur Herstellung von Digoxigenin-Derivatender allgemeinen Formel (I)

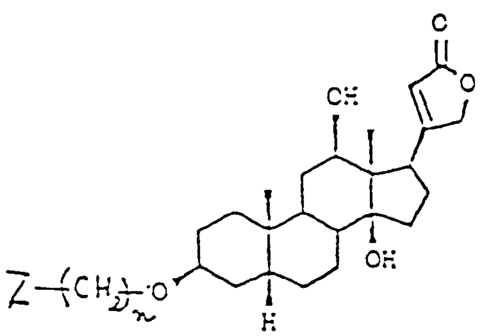

(I)

worin n eine ganze Zahl von 1 bis 4 darstellt, und Z die Gruppe -COOH, -CN, $-COOR^5$, $-CONR^1R^2$, $-COONR^3R^4$, $-CON_3$, $-COOCOO-R^5$ oder $-COOCO-R^5$ bedeutet, worin $R^1$, $R^2$, $R^3$ und $R^4$ unabhängig voneinander ein Wasserstoffatom, eine Alkylgruppe, eine Aralkylgruppe oder eine Arylgruppe sind, oder $R^1$ und $R^2$ oder $R^3$ und $R^4$ zusammen auch ein carbo- oder heterocyclisches Ringsystem bilden können, und $R^5$ eine Alkyl- oder Aralkylgruppe ist,
**dadurch gekennzeichnet,**
daß man im 12-O-Acetyl-digoxigenin die frei OH-Gruppe in 3-Stellung durch Umsetzung mit Diazoessigester in an sich bekannter Weise in eine Verbindung der Formel (I) überführt, worin Z = $COOC_2H_5$ ist, und, wenn erwünscht, in dem erhaltenen Reaktionsprodukt in an sich bekannter Weise die $COOC_2H_5$-Gruppe in die COOH-Gruppe oder in eine der anderen Gruppen überführt, die Z darstellen kann und, wenn erwünscht, in den so erhaltenen Reaktionsprodukten die Gruppe Z in an sich bekannter Weise in eine andere Gruppe Z gemäß Formel (I) überführt.

2. Verfahren nach Anspruch 1,
**dadurch gekennzeichnet,**
daß Z bedeutet: -COOH, $-COOC_2H_5$,

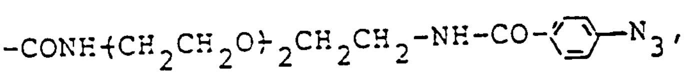,
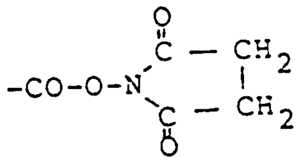

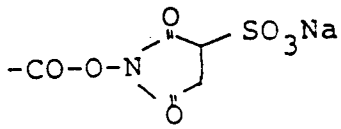,

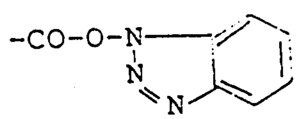,
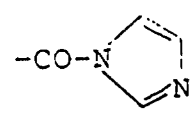

3. Verfahren nach Anspruch 1 oder 2,
**dadurch gekennzeichnet,**
daß n = 1 ist.

4. Verfahren nach einem der Ansprüche 1 bis 3,
**dadurch gekennzeichnet,**
daß man ein Derivat der Formel I mit einem Trägermaterial umsetzt, welches ein immunogenes Trägermaterial, eine Nukleinsäure oder die markierte Struktureinheit eines markierten Digoxigenin-Konjugates für die Verwendung in Immunoassays darstellt, unter Bildung eines Konjugats der Formel II,

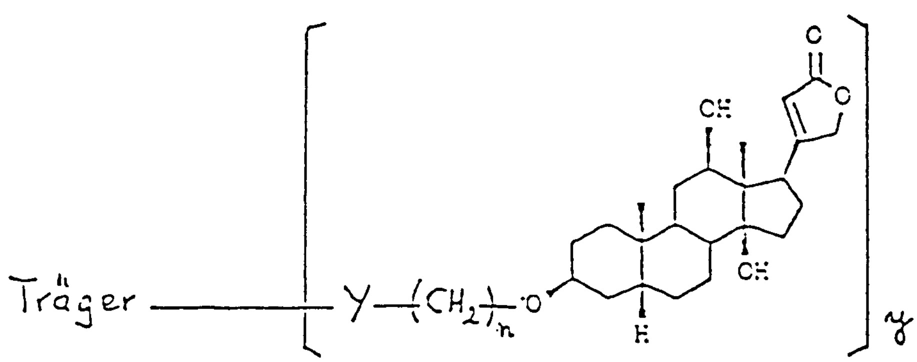
(II)

in welcher y im Durchschnitt eine Zahl von 1 bis 20 bedeutet, und Y die durch Umsetzung der Carboxylfunktion Z der Digoxigenin-Derivate der Formel (I) nach einem der Ansprüche 1 bis 3 und den reagierenden Stellen des Trägermaterials gebildete Gruppierung ist.

5. Verwendung der Digoxigenin-Derivate der Formel (I) gemäß einem der Ansprüche 1 bis 3 für die Herstellung von markierten Konjugaten (labeled conjugates) zur Bestimmung von kardiotonischen Glycosiden, insbesondere von Digoxin, in Immunoassays.

6. Verwendung der Digoxigenin-Derivate der Formel (I) gemäß einem der Ansprüche 1 bis 3 als Markerhapten zum Nachweis von Nucleinsäuren durch Hybridisierung mit einer komplementären Nucleinsäure-Sonde, die über eine chemische Verbindung mindestens ein Hapten als Markierung gebunden enthält.

7. Verwendung der Digoxigenin-Konjugate der Formel (11) nach Anspruch 4, worin der Träger ein immunogenes Trägermaterial darstellt, zur Immunisierung von zur Antikörper-Bildung geeigneten

Organismen.

8. Verwendung der Digoxigenin-Konjugate der Formel (11) nach Anspruch 4, worin der Träger die markierte Struktureinheit eines markierten Digoxigenin-Konjugates darstellt, in Immunoassays zur Bestimmung von kardiotonischen Glycosiden, insbesondere von Digoxin.

**Claims**

**Claims for the following Contracting States : AT, BE, CH, DE, FR, GB, GR, IT, LI, LU, NI, SE**

1. Digoxigenin derivatives of the general formula (I)

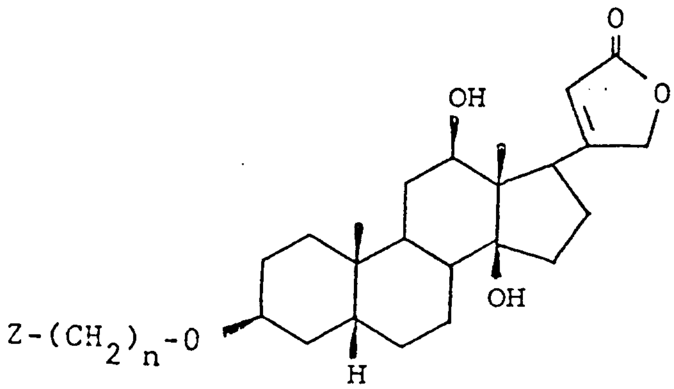

(I)

wherein n represents a whole number from 1 to 4 and Z signifies the group -COOH, -CN, $-COOR^5$, $-CONR^1R^2$, $-COONR^3R^4$, $-CON_3$, $-COOCOO-R^5$ or $-COOCO-R^5$, wherein $R^1$, $R^2$, $R^3$ and $R^4$, independently of one another, are a hydrogen atom, an alkyl group, an aralkyl group or an aryl group or $R^1$ and $R^2$ or $R^3$ and $R^4$ together can also form a carbo- or heterocyclic ring system and $R^5$ is an alkyl or aralkyl group.

2. Digoxigenin derivatives of the formula (I) according to claim 1, characterised in that Z signifies -COOH, $-COOC_2H_5$,

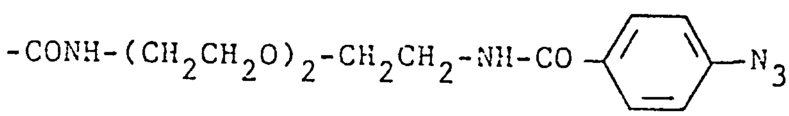 ,

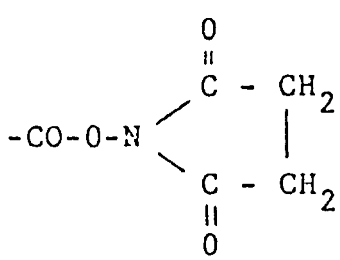 , 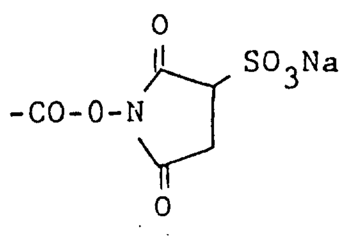

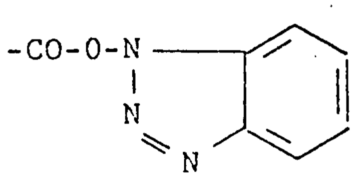 , 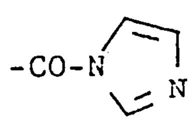

**3.** Digoxigenin derivatives of the formula (I) according to one of claims 1 or 2, characterised in that n = 1.

**4.** Process for the preparation of digoxigenin derivatives of the formula (I) according to one of claims 1 to 3, characterised in that, in the 12-O-acetyldigoxigenin, one converts the free OH group in the 3-position by reaction with diazoacetic acid ester in per se known manner into a compound of the formula (I), wherein Z = $-COOC_2H_5$ and, if desired, in the reaction product obtained, converts the $-COOC_2H_5$ group into the -COOH group or into one of the other groups which can represent Z and, if desired, in the so-obtained reaction products, converts the group Z in per se known manner into another group Z according to formula (I).

**5.** Use of the digoxigenin derivatives of the formula (I) according to one of claims 1 to 3 for the preparation of labelled conjugates for the determination of cardiotonic glycosides, especially of digoxin, in immunoassays.

**6.** Use of the digoxigenin derivatives of the formula (I) according to one of claims 1 to 3 as labelling haptens for the detection of nucleic acids by hybridisation with a complementary nucleic acid probe which, via a chemical bond, contains bound at least one hapten as label.

**7.** Digoxigenin conjugates according to formula (II)

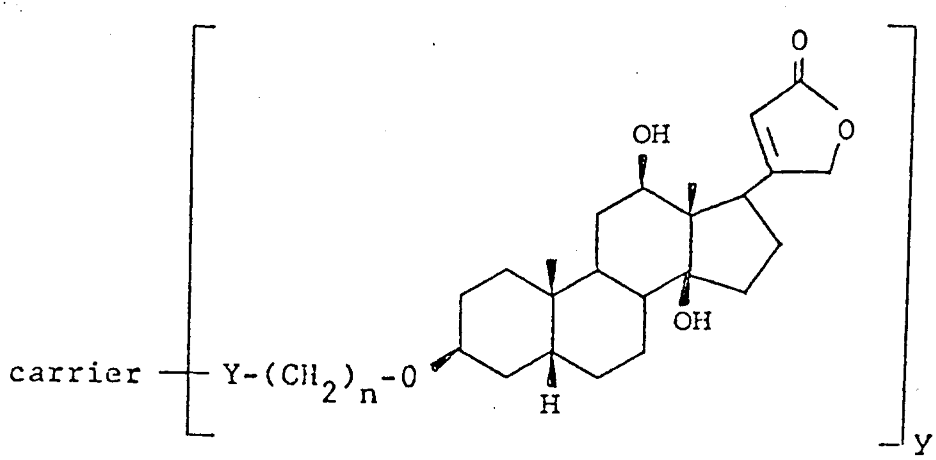

(II)

wherein the carrier signifies an immunogenic carrier material, a nucleic acid or the labelled structural unit of a labelled digoxigenin conjugate for the use in immunoessays, y signifies, on average, a number from 1 to 20 and Y is the grouping formed by reaction of the carboxyl function Z of the digoxigenin derivative of the formula (I) according to one of claims 1 to 3 and the reacting positions of the carrier material.

**8.** Use of the digoxigenin conjugates of the formula (II) according to claim 7, wherein the carrier represents an immunogenic carrier material, for the immunisation of organisms suitable for antibody formation.

**9.** Use of the digoxigenin conjugates of the formula (II) according to claim 7, wherein the carrier represents the labelled structural unit of a labelled digoxigenin conjugate, in immunoassays for the determination of cardiotonic glycosides, especially of digoxin.

**Claims for the following Contracting State : ES**

**1.** Process for the preparation of digoxigenin derivatives of the general formula I

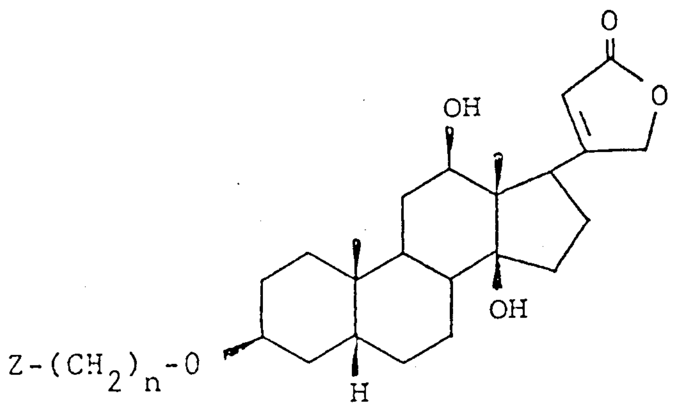

(I)

wherein n is a whole number from 1 to 4 and Z signifies the group -COOH, -CN, $-COOR^5$, $-CONR^1R^2$, $-COONR^3R^4$, $-CON_3$, $-COOCOO-R^5$ or $-COOCO-R^5$, wherein $R^1$, $R^2$, $R^3$ and $R^4$, independently of one another, are a hydrogen atom, an alkyl group, an aralkyl group or an aryl group or $R^1$ and $R^2$ or $R^3$ and $R^4$ together can also form a carbo- or heterocyclic ring system and $R^5$ is an alkyl or aralkyl group, characterised in that, in the 12-O-acetyldigoxigenin, one converts the free OH group in the 3-position by reaction with diazoacetic acid ester in per se known manner into a compound of the formula (I), wherein Z = $-COOC_2H_5$ and, if desired, in the reaction product obtained, converts the $-COOC_2H_5$ group into the -COOH group or into one of the other groups which can represent Z and, if desired, in the so obtained reaction products, converts the group Z in per se known manner into another group according to formula (I).

**2.** Process according to claim 1, characterised in that Z signifies -COOH, $-COOC_2H_5$,

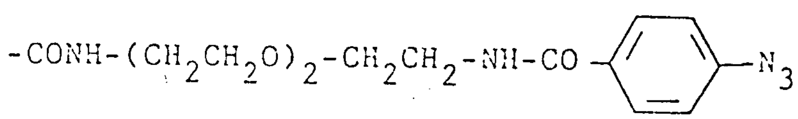 ,

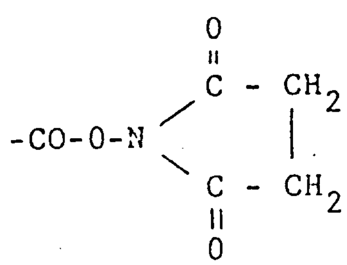 , 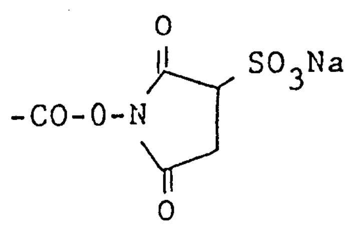

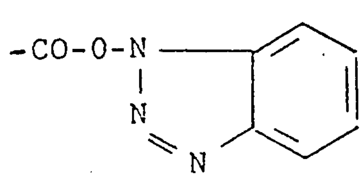 , 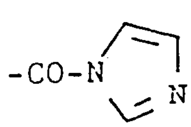

3. Process according to claim 1 or 2, characterised in that n = 1.

4. Process according to one of claims 1 to 3, characterised in that one reacts a derivative of the formula I with a carrier material which represents an immunogenic carrier material, a nucleic acid or the labelled structural unit of a labelled digoxigenin conjugate for the use in immunoassays, with the formation of a conjugate of the formula II,

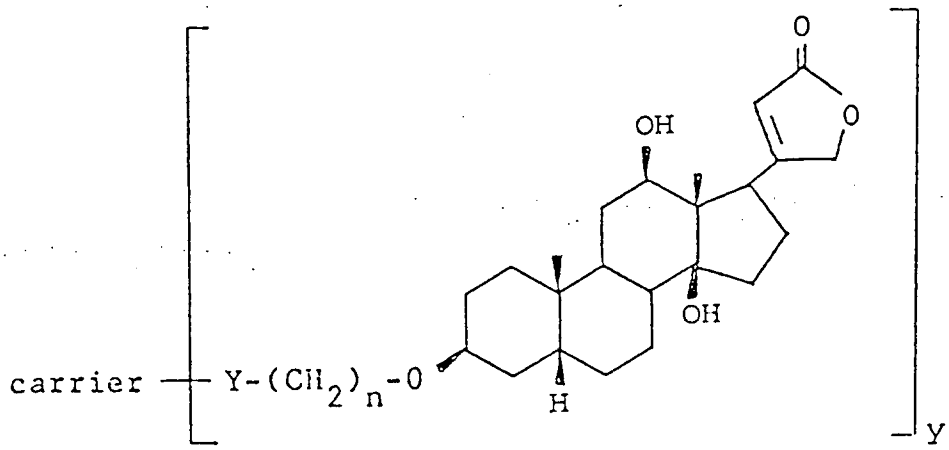

(II)

in which y, on average, signifies a number from 1 to 20 and Y is the grouping formed by reaction of the carboxyl function Z of the digoxigenin derivative of the general formula I according to one of claims 1 to 3 and the reacting positions of the carrier material.

5. Use of the digoxigenin derivatives of the formula (I) according to one of claims 1 to 3 for the preparation of the labelled conjugates for the determination of cardiotonic glycosides, especially of digoxin, in immunoassays.

6. Use of the digoxigenin derivatives of the formula (I) according to one of claims 1 to 3 as labelling hapten for the detection of nucleic acids by hybridisation with a complementary nucleic acid probe which contains at least one hapten as labelling bound via a chemical bond.

7. Use of digoxigenin conjugates of the formula (II) according to claim 4, wherein the carrier represents an immunogenic carrier material, for the immunisation of organisms suitable for antibody formation.

8. Use of digoxigenin conjugates of the formula (II) according to claim 4, wherein the carrier represents the labelled structural unit of a labelled digoxigenin conjugate, in immunoassays for the determination of cardiotonic glycosides, especially of digoxin.

## Revendications

**Revendications pour les Etats contractants suivants : AT, BE, CH, DE, FR, GB, GR, IT, LI, LU, NL, SE**

1. Dérivés de la digoxigénine de formule générale (I)

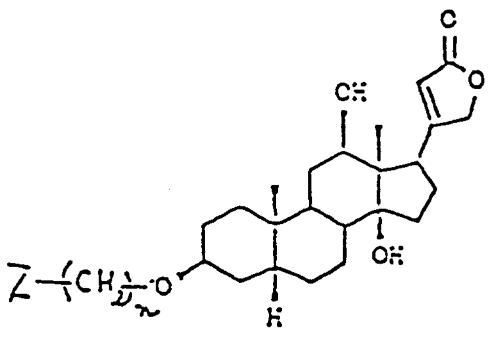

(I)

dans laquelle n représente un nombre entier de 1 à 4, et Z représente le groupe -COOH, -CN, $-COOR^5$, $-CONR^1R^2$, $-COONR^3R^4$, $-CON_3$, $-COOCOO-R^5$ ou $-COOCO-R^5$, où $R^1$, $R^2$, $R^3$ et $R^4$ représentent indépendamment les uns des autres un atome d'hydrogène, un groupe alkyle, un groupe aralkyle ou un groupe aryle, ou bien $R^1$ et $R^2$ ou $R^3$ et $R^4$ peuvent aussi former ensemble un système cyclique carbocyclique ou hétérocyclique, et $R^5$ représente un groupe alkyle ou aralkyle.

2. Dérivés de la digoxigénine de formule (I) selon la revendication 1, caractérisés en ce que Z signifie: -COOH, $-COOC_2H_5$,

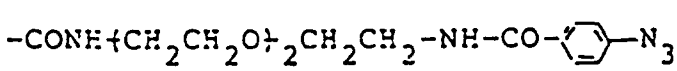, 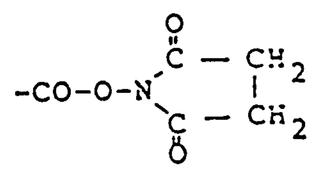

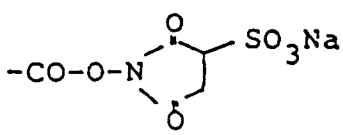,

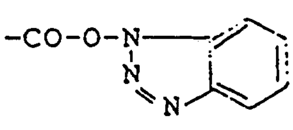, 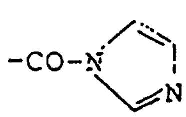

3. Dérivés de la digoxigénine de formule (I) selon l'une des revendications 1 ou 2, caractérisés en ce que n = 1.

4. Procédé de préparation des dérivés de la digoxigénine de formule (I) selon l'une des revendications 1 à 3, caractérisé en ce que, dans la 12-O-acétyl-digoxigénine, on convertit le groupe OH libre en position 3 par réaction avec le diazoacétate d'éthyle de manière connue en soi en un composé de formule (I) où Z = $COOC_2H_5$, et, lorsqu'on le souhaite, on convertit de manière connue en soi dans le produit réactionnel obtenu le groupe $COOC_2H_5$ en le groupe COOH ou en l'un des autres groupes que Z peut représenter et, lorsqu'on le souhaite, dans les produits réactionnels ainsi obtenus on convertit de manière connue en soi le groupe Z en un autre groupe Z selon la formule (I).

5. Utilisation des dérivés de la digoxigénine de formule (I) selon l'une des revendications 1 à 3 pour la préparation de conjugués marqués (labeled conjugates) pour la détermination de glycosides cardiotoniques, en particulier de la digoxine, dans des immuno-analyses.

6. Utilisation des dérivés de la digoxigénine de formule (I) selon l'une des revendications 1 à 3 comme haptène marqueur pour la mise en évidence d'acides nucléiques par hybridation avec une sonde d'acide nucléique complémentaire qui contient comme marquage au moins un haptène lié par l'intermédiaire d'une combinaison chimique.

7. Conjugués de digoxigénine selon la formule (II)

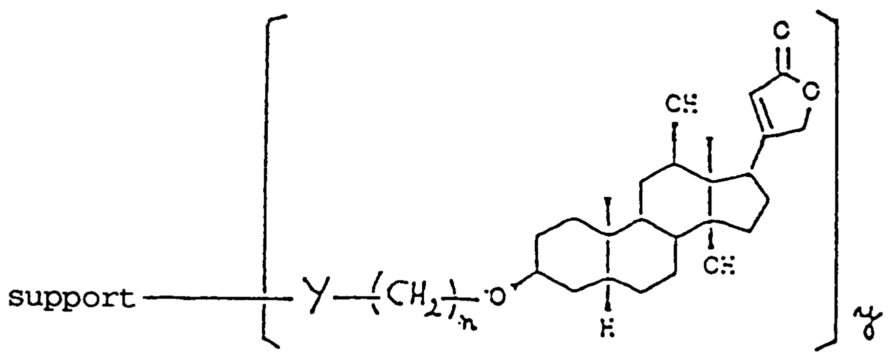

(II)

dans laquelle le support représente un matériau support immunogène, un acide nucléique ou l'unité structurale marquée d'un conjugué de digoxigénine marqué destiné à être utilisé dans des immuno-analyses, y représente en moyenne un nombre de 1 à 20, et Y est le groupement formé par réaction de la fonction carboxyle Z des dérivés de la digoxigénine de formule (I) selon l'une des revendications 1 à 3 et des sites réagissants du matériau support.

8. Utilisation des conjugués de digoxigénine de formule (II) selon la revendication 7, où le support représente un matériau support immunogène, pour l'immunisation d'organismes convenant pour la formation d'anticorps.

9. Utilisation des conjugués de digoxigénine de formule (II) selon la revendication 7, où le support représente l'unité structurale marquée d'un conjugué de digoxigénine marqué, dans des immuno-analyses pour la détermination de glycosides cardiotoniques, en particulier de la digoxine.

**Revendications pour l'Etat contractant suivant : ES**

1. Procédé de préparation de dérivés de la digoxigénine de formule générale (I)

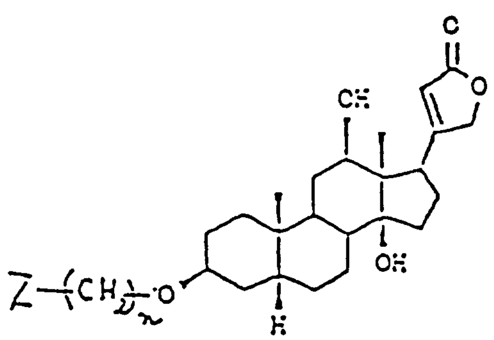

(I)

dans laquelle n représente un nombre entier de 1 à 4, et Z représente le groupe -COOH, -CN, $-COOR^5$, $-CONR^1R^2$, $-COONR^3R^4$, $-CON_3$, $-COOCOO-R^5$ ou $-COOCO-R^5$, où $R^1$, $R^2$, $R^3$ et $R^4$

représentent indépendamment les uns des autres un atome d'hydrogène, un groupe alkyle, un groupe aralkyle ou un groupe aryle, ou bien $R^1$ et $R^2$ ou $R^3$ et $R^4$ peuvent aussi former ensemble un système cyclique carbocyclique ou hétérocyclique, et $R^5$ représente un groupe alkyle ou aralkyle, caractérisé en ce que, dans la 12-O-acétyl-digoxigénine, on convertit le groupe OH libre en position 3 par réaction avec le diazoacétate d'éthyle de manière connue en soi en un composé de formule (I) où Z = $COOC_2H_5$, et, lorsqu'on le souhaite, on convertit de manière connue en soi dans le produit réactionnel obtenu le groupe $COOC_2H_5$ en le groupe COOH ou en l'un des autres groupes que Z peut représenter et, lorsqu'on le souhaite, dans les produits réactionnels ainsi obtenus on convertit de manière connue en soi le groupe Z en un autre groupe Z selon la formule (I).

2. Procédé selon la revendication 1, caractérisé en ce que Z signifie: -COOH, $-COOC_2H_5$,

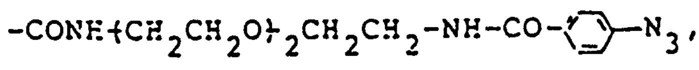, 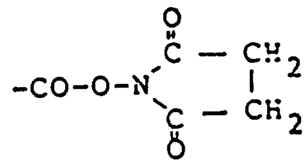

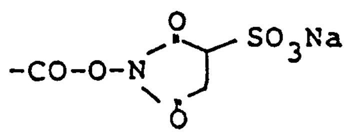,

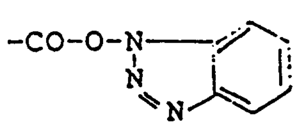, 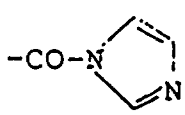

3. Procédé selon la revendication 1 ou 2, caractérisé en ce que n = 1.

4. Procédé selon l'une des revendications 1 à 3, caractérisé en ce que l'on fait réagir un dérivé de formule I avec un matériau support qui représente un matériau support immunogène, un acide nucléique ou l'unité structurale marquée d'un conjugué de digoxigénine marqué destiné à être utilisé dans des immuno-analyses, avec formation d'un conjugué de formule II

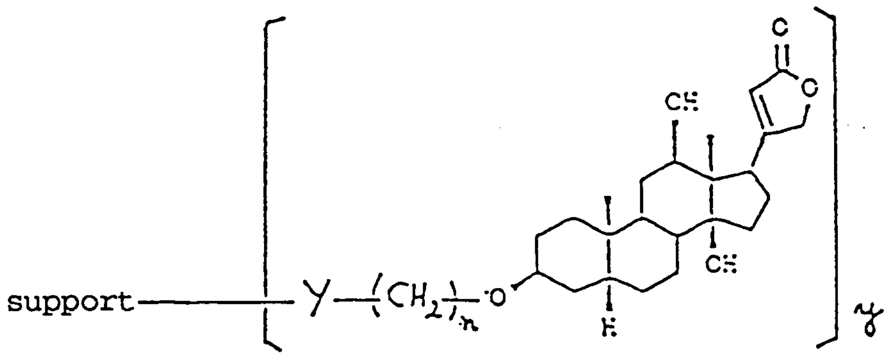

(II)

dans laquelle y représente en moyenne un nombre de 1 à 20, et Y est le groupement formé par réaction de la fonction carboxyle Z des dérivés de la digoxigénine de formule (I) selon l'une des revendications 1 à 3 et des sites réagissants du matériau support.

5. Utilisation des dérivés de la digoxigénine de formule (I) selon l'une des revendications 1 à 3 pour la préparation de conjugués marqués (labeled conjugates) pour la détermination de glycosides cardiotoniques, en particulier de la digoxine, dans des immuno-analyses.

**6.** Utilisation des dérivés de la digoxigénine de formule (I) selon l'une des revendications 1 à 3 comme haptène marqueur pour la mise en évidence d'acides nucléiques par hybridation avec une sonde d'acide nucléique complémentaire qui contient comme marquage au moins un haptène lié par l'intermédiaire d'une combinaison chimique.

**7.** Utilisation des conjugués de digoxigénine de formule (II) selon la revendication 4, où le support représente un matériau support immunogène, pour l'immunisation d'organismes convenant pour la formation d'anticorps.

**8.** Utilisation des conjugués de digoxigénine de formule (II) selon la revendication 4, où le support représente l'unité structurale marquée d'un conjugué de digoxigénine marqué, dans des immuno-analyses pour la détermination de glycosides cardiotoniques, en particulier de la digoxine.